# EUROPÄISCHE PATENTANMELDUNG

(11) **EP 4 335 839 A1**
(43) Veröffentlichungstag der Anmeldung: **13.03.2024**
(21) Anmeldenummer: 22194934.0
(22) Anmeldetag: 09.09.2022
(51) Int. Cl.: C07C 263/10, C07C 263/18, C07C 265/14

(54) **VERFAHREN ZUR BEHANDLUNG VON RÜCKSTÄNDEN AUS DER PRODUKTION ORGANISCHER ISOCYANATE**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft ein Verfahren zur Behandlung von Rückständen aus der Produktion organischer Isocyanate durch Phosgenierung organischer Amine, umfassend einen Vermischungsschritt eines ersten Rückstands A mit einem zweiten Rückstand B unter Erhalt einer Rückstandsmischung, wobei der Rückstand A aus der Produktion von 1,5-Pentandiisocyanat erhalten wurde und der Rückstand B aus der Produktion eines von 1,5-Pentandiisocyanat verschiedenen Isocyanats stammt. Sie betrifft außerdem die Verwendung eines ersten Rückstands A aus der Produktion von 1,5-Pentandiisocyanat zur Stabilisierung eines zweiten Rückstands B aus der Produktion mindestens eines von 1,5-Pentandiisocyanat verschiedenen, organischen Isocyanats sowie stabilisierte Rückstandsmischungen.

## Beschreibung

Die vorliegende Erfindung betrifft ein Verfahren zur Behandlung von Rückständen aus der Produktion organischer Isocyanate durch Phosgenierung organischer Amine. Sie betrifft außerdem die Verwendung eines Rückstands aus der Produktion von 1,5-Pentandiisocyanat sowie stabilisierte Rückstandsmischungen.

Die großtechnische Herstellung von Isocyanaten durch Umsetzung von Aminen mit Phosgen ist bekannt und in der Literatur ausführlich beschrieben (z.B. Ullmanns Encyklopädie der technischen Chemie, 4. Auflage, Band 13, Seite 347-357, Verlag Chemie, GmbH, D-6940 Weinheim, 1977 oder auch EP 1 575 908 A1). Zumeist fällt bei der Herstellung reiner destillierter Diisocyanate in den Destillationsprozessen ein Nebenproduktstrom an, der nach weitmöglicher destillativer Abtrennung von freien Isocyanaten als Rückstand entsorgt werden muss.

Solche Rückstände enthalten stets noch reaktive Gruppen und es besteht deshalb die Gefahr exothermer Reaktionen, bis hin zum thermischen Durchgehen (thermal runaway), also der unkontrollierten Überhitzung der exothermen chemischen Reaktion und der technischen Apparatur, in der der Rückstand gelagert wird. Um dies zu vermeiden, werden Untersuchungen zur thermischen Stabilität der Rückstände durchgeführt, mit dem Ziel, die freiwerdende Reaktionswärme und die sogenannte Onset-Temperatur zu ermitteln, also die Temperatur, ab der die Reaktion startet und Energie freisetzt. Der Rückstand darf dann nur für eine gewisse Zeit und bei Temperaturen gelagert werden, die um einen Sicherheitsabstand niedriger liegen als die Onset-Temperatur.

Da es vorteilhaft ist, Rückstände in flüssiger Form zu handhaben und die Erstarrungspunkte mit zunehmender Konzentration der Schwersiedenden Nebenprodukte im Rückstand ansteigen, müssen bei niedrigeren Onset-Temperaturen und entsprechend niedrigeren zulässigen Rückstandstemperaturen, die Rückstände stärker verdünnt sein. Es bleibt also entweder mehr des Wertprodukts im Rückstand oder es müssen zusätzliche Maßnahmen ergriffen werden, wie etwa die Zugabe von Lösungsmitteln.

Um unerwartete Reaktionen im Rückstand zu vermeiden, ist es üblicherweise erstrebenswert, verschiedenartige Rückstände separat zu handhaben und nicht zu vermischen.

Daher war es Aufgabe der vorliegenden Erfindung ein Verfahren bereitzustellen, welches nicht mit den vorgenannten Nachteilen aus dem Stand der Technik behaftet ist und ein verbessertes Handling von Rückstandsmischungen aus der Produktion organischer Isocyanate ermöglicht.

Die Aufgabe wurde erfindungsgemäß gelöst, durch ein Verfahren zur Behandlung von Rückständen aus der Produktion organischer Isocyanate durch Phosgenierung organischer Amine, umfassend einen Vermischungsschritt eines ersten Rückstands A mit einem zweiten Rückstand B unter Erhalt einer Rückstandsmischung, wobei der Rückstand A aus der Produktion von 1,5-Pentandiisocyanat erhalten wurde und der Rückstand B aus der Produktion eines von 1,5-Pentandiisocyanat verschiedenen Isocyanats stammt.

Überraschend wurde nun gefunden, dass durch die Beimischung eines Rückstands, bevorzugt eines Destillationsrückstands, aus der Produktion von 1,5-Pentandiisocyanat (im Folgenden auch als Pentamethylendiisocyanat oder auch als PDI bezeichnet) zu anderen Rückständen aus der Produktion von Isocyanaten, die von Pentamethylendiisocyanat verschieden sind, die Gefahr einer unkontrollierten exothermen Reaktion nicht erhöht, sondern sogar häufig verringert wird. Dies ermöglicht also die Vermischung derartiger Rückstände, ohne die Prozesssicherheit zu verringern, wodurch sich positive technische Effekte erzielen lassen, wie beispielsweise eine längere Lagerstabilität des gemischten Rückstands oder ein reduzierter apparativer Aufwand durch Einsparung zusätzlicher Rückstandsvorlagen.

Bevorzugt bedeuten erfindungsgemäß die Ausdrücke "umfassend" oder "enthaltend", "im Wesentlichen bestehend aus" und besonders bevorzugt "bestehend aus". Die in den Patentansprüchen und in der Beschreibung genannten weiteren Ausführungsformen können beliebig kombiniert werden, sofern sich aus dem Kontext nicht eindeutig das Gegenteil ergibt.

Eine "organische Verbindung" oder "organischer Rest" enthält mindestens eine Einheit, die eine kovalente Kohlenstoff-Wasserstoff-Bindung umfasst.

Vorliegend ist der Begriff "aliphatisch" definiert als nicht-aromatische Kohlenwasserstoffgruppen, die gesättigt oder ungesättigt sind.

Vorliegend ist der Begriff "araliphatisch" definiert als Kohlenwasserstoffreste, die sowohl aus einer aromatischen als auch aus einer gesättigten oder ungesättigten Kohlenwasserstoffgruppe bestehen, die direkt an den aromatischen Rest gebunden ist.

Vorliegend ist der Begriff "alicyclisch" oder "cycloaliphatisch" definiert als gegebenenfalls substituierte, carbocyclische oder heterocyclische Verbindungen oder Einheiten, die nicht aromatisch sind.

"Mindestens ein", wie hierin verwendet, bezieht sich auf 1 oder mehr, beispielsweise 2, 3, 4, 5, 6, 7, 8, 9 oder mehr. Im Zusammenhang mit Bestandteilen der hierin beschriebenen Verbindungen bezieht sich diese Angabe nicht auf die absolute Menge an Molekülen, sondern auf die Art des Bestandteils. "mindestens eines von Pentamethylendiisocyanat verschiedenen Isocyanats" bedeutet daher beispielsweise, dass nur eine Art von Verbindung oder mehrere verschiedene Arten von Verbindungen dieser Art, ohne Angaben über die Menge der einzelnen Verbindungen zu machen, enthalten sein können.

Numerische Bereiche, die in dem Format "in/von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Das erfindungsgemäße Verfahren lässt sich beispielsweise dann vorteilhaft einsetzen, wenn in einer Produktionsanlage zur Herstellung von organischen Isocyanaten Kampagnen gefahren werden, in denen jeweils unterschiedliche organische Isocyanate hergestellt werden, wobei eines der organischen Isocyanate Pentamethylendiisocyanat ist. Bei der Herstellung oder der Aufreinigung der verschiedenen organischen Isocyanate fallen dann der Rückstand A und der zweite Rückstand B zeitlich versetzt zueinander an und können beispielsweise in einer Rückstandsvorlage gemischt werden, um die gewünschten Effekte, insbesondere eine Stabilisierung, zu erzielen. Es müssen also nicht für jeden artverschiedenen Rückstand eigene Rückstandsvorlagen vorhanden sein oder, sofern lediglich eine Rückstandsvorlage verfügbar ist, muss diese nicht beim Kampagnenwechsel komplett geleert oder gar gespült werden. Vielmehr bietet es sich an, stets eine gewisse Menge an Rückstandsmischung vorzuhalten, um die jeweiligen Rückstände in der Destillation optimal aufkonzentrieren zu können und von der Stabilisierung durch die Mischung mit 1,5-Pentandiisocyanat basiertem Rückstand zu profitieren.

Eine andere Möglichkeit bietet sich, wenn zwei Produktions- und/oder Destillationsvorrichtungen für die verschiedenen organischen Isocyanate an einem Standort vorhanden sind. In diesem Fall fallen der Rückstand A und der Rückstand B zeitgleich an und können entsprechend dem erfindungsgemäßen Verfahren dem Vermischungsschritt unterzogen werden. Dieser kann entweder in einer Rohrleitung erfolgen, über die die Rückstände in eine Sammelvorlage, einen Transportbehälter oder zu einer Verbrennungsanlage überführt werden oder er erfolgt in einer Rückstandsvorlage. Zur Mischung in der Rohrleitung können die Rückstände einfach in einer Rohrleitung vereint werden. Vorzugsweise wird die Vermischung der Rückstände A und B mit Hilfe eines Statikmischers oder eines dynamischen Mischaggregats wie beispielsweise einer oder mehrerer Mischpumpen, Dispergierer oder Rührbehälter verbessert. Erfolgt die Mischung in einer Sammelvorlage, so hat diese vorzugsweise jeweils einen eigenen Einlassstutzen, gegebenenfalls mit einem Tauchrohr, für die verschiedenen Rückstände. Die Mischung erfolgt dann in der Sammelvorlage mit Hilfe eines Rührwerks und/oder einer Umpumpung, die auch zur Temperierung des Rückstands genutzt werden kann. Bevorzugt erfolgt die Vermischung mit Hilfe eines Rührwerks in der Sammelvorlage.

Beim Rückstand A handelt es sich um einen flüssigen Rückstand oder eine Suspension, vorzugsweise einen flüssigen Rückstand, der in der Produktion von Pentamethylendiisocyanat erhalten wurde und der neben PDI beispielsweise auch Verbindungen enthält, die einen höheren Siedepunkt als PDI aufweisen und auf Basis von PDI entstanden sind. Dies können beispielsweise PDI-Oligomere wie Carbodiimide oder Trimere, Harnstoffe, Urethane, aber auch chlorierte Verbindungen wie Carbamoylchlorid sein. Bevorzugt handelt es sich bei dem Rückstand A um einen vorzugsweise flüssigen Destillationsrückstand, der am Sumpf einer Kolonne erhalten wurde, in der PDI aus einem PDIenthaltenden Stoffgemisch abgetrennt wurde. Vorliegend wird ein solcher Rückstand A auch als "aus der Produktion von Pentamethylendiisocyanat" bezeichnet.

Beim Rückstand B handelt es sich um einen flüssigen Rückstand oder eine Suspension, vorzugsweise einen flüssigen Rückstand, der in der Produktion mindestens eines von Pentamethylendiisocyanat verschiedenen Isocyanats erhalten wurde und der neben dem mindestens einen Isocyanat beispielsweise auch Verbindungen enthält, die auf Basis des mindestens einen Isocyanats entstanden sind und einen höheren Siedepunkt als das jeweilige Isocyanat aufweisen. Vorzugsweise handelt es sich um einen Rückstand aus der Produktion von genau einem von PDI verschiedenen Isocyanat. Bevorzugt handelt es sich bei dem mindestens einen von PDI verschiedenen Isocyanat, in dessen Herstellungs- und/oder Reinigungsprozess der Rückstand B anfällt, um mindestens ein aliphatisches, cycloaliphatisches und/oder araliphatisches Diisocyanat, besonders bevorzugt um mindestens ein cycloaliphatisches und/oder araliphatisches Diisocyanat. Weiter bevorzugt handelt es sich bei dem mindestens einen von PDI verschiedenen Isocyanat um Hexamethylendiisocyanat, Diisocyanatohexylmethan, Isophorondiisocyanat (IPDI), Bis-(isocyanatomethyl)-norbornan (NBDI), 1,3- und 1,4-Bis-(isocyanatomethyl)-cyclohexan und/oder Xylylyendiisocyanat, besonders bevorzugt handelt es sich um Hexamethylendiisocyanat, Diisocyanatohexylmethan, Isophorondiisocyanat (IPDI) und/oder Xylylyendiisocyanat, ganz besonders bevorzugt handelt es sich um IPDI und/oder Xylylendiisocyanat und am meisten bevorzugt um IPDI und/oder m-Xylylendiisocyanat (im Folgenden auch als XDI bezeichnet). Vorliegend wird ein solcher Rückstand B auch als "aus der Produktion mindestens eines von Pentamethylendiisocyanat verschiedenen Isocyanats" bezeichnet. Bevorzugt handelt es sich bei dem mindestens einen von PDI verschiedenen Isocyanat um genau ein von PDI verschiedenes Isocyanat und gegebenenfalls dessen Isomere und damit auch um einen Rückstand, der nur auf diesem einen von PDI verschiedenen Isocyanat sowie gegebenenfalls dessen Isomeren basiert.

Der Herstellungsprozess, also die Synthese an sich, und der Reinigungsprozess sind beides Schritte, die vorliegend zur Produktion eines Isocyanats gezählt werden, so dass der Begriff Produktion eines Isocyanats sich vorliegend nicht nur auf die Herstellung an sich bezieht, sondern ein Rückstand aus der Produktion eines Isocyanats ebenso auch in der Destillation angefallen sein kann.

In einer weiteren Ausführungsform des erfindungsgemäßen Verfahrens sind der Rückstand A und/oder der Rückstand B, bevorzugt der Rückstand A und der Rückstand B, Destillationsrückstände.

In einer bevorzugten Ausführungsform stammt der Rückstand B aus der Produktion genau eines von 1,5-Pentandiisocyanat verschiedenen aliphatischen, cycloaliphatischen oder araliphatischen Diisocyanats, vorzugsweise genau eines araliphatischen oder cycloaliphatischen Diisocyanats. Besonders bevorzugt stammt der Rückstand B aus der Produktion von Hexamethylendiisocyanat, Diisocyanatohexylmethan, IPDI, Bis-(isocyanatomethyl)-norbornan (NBDI), 1,3- und 1,4-Bis-(isocyanatomethyl)-cyclohexan oder Xylylendiisocyanat, ganz besonders bevorzugt aus der Produktion von IPDI oder Xylylendiisocyanat und am meisten bevorzugt aus der Produktion von IPDI oder XDI.

Um die Ausbeute an Wertprodukt und damit die Wirtschaftlichkeit der Produktionsverfahren zu maximieren, ist es wünschenswert, dass der Rückstand A und/oder der Rückstand B möglichst wenig des entsprechenden Wertprodukts enthalten. Ein zu geringer Restgehalt an monomerem Isocyanat führt jedoch zu einem starken Viskositätsanstieg bis hin zur Verfestigung des Rückstands, so dass in der Praxis stets ein Kompromiss gefunden werden muss. Üblicherweise enthalten die Rückstände noch ca. 5 bis 85 Gew.-%, bevorzugt 10 bis 60 Gew.-%, besonders bevorzugt 15 bis 50 Gew.-% und ganz besonders bevorzugt 20 bis 40 Gew.-% des monomeren Isocyanats, wenn sie aus der Produktion des Isocyanats entnommen werden. Prinzipiell kann die Temperatur beliebig gewählt werden. Eine höhere Temperatur des Rückstands hilft dabei, die Viskosität gering zu halten und damit die Pumpfähigkeit zu erhalten. Einer Temperaturerhöhung steht allerdings entgegen, dass die Inhaltsstoffe der Isocyanat-Rückstände miteinander bzw. mit sich selbst exotherme Reaktionen eingehen können. Eine Temperatur im Bereich von 15 °C bis 180 °C, bevorzugt im Bereich von 40 °C bis 150 °C und besonders bevorzugt im Bereich von 60 °C bis 110 °C hat sich als vorteilhafter Kompromiss erwiesen.

Dementsprechend ist es auch bevorzugt, dass im Vermischungsschritt zumindest zeitweise, bevorzugt durchgehend eine Temperatur im Bereich von 15 °C bis 180 °C, bevorzugt im Bereich von 40 °C bis 150 °C und besonders bevorzugt im Bereich von 60 °C bis 110 °C vorliegt.

Um Temperatur- und Konzentrationsschwankungen zu verringern, ist es bevorzugt, das mindestens eine Rückstandsgemisch, welches durch den Vermischungsschritt erhalten wird, in mindestens einem Rückstandsbehälter zu lagern, wobei der Vermischungsschritt in einer separaten, dem Rückstandsbehälter vorgelagerten Mischvorrichtung oder vorzugsweise in dem Rückstandsbehälter selbst erfolgen kann. Die im Rückstandsbehälter vorliegende Menge an Rückstandsgemisch puffert auf diese Weise Schwankungen im neu zulaufenden Rückstandsstrom ab.

Zuvor genannte Überlegungen bezüglich der bevorzugten Temperaturen im Vermischungsschritt lassen sich auch auf eine Lagerung des im Vermischungsschritt erhaltenen mindestens einen Rückstandsgemisches übertragen. Entsprechend erfolgt die Lagerung zumindest zeitweise, bevorzugt durchgehend bei einer Temperatur im Bereich von 15 °C bis 180 °C, bevorzugt im Bereich von 40 °C bis 150 °C. und besonders bevorzugt im Bereich von 60 °C bis 110 °C erfolgt. Gerade bei längerer Lagerung des erhaltenen Rückstandsgemisches ist es wichtig, die Temperaturgrenzen einzuhalten, da die Wahrscheinlichkeit nachteiliger Effekte wie einer Verfestigung des Rückstands oder einer exothermen Zersetzung mit längerer Lagerungsdauer steigt. Sofern eine Lagerung des Rückstands erfolgt, ist es deshalb vorteilhaft, die Verweilzeit des Rückstands in dem Lagerbehälter zu begrenzen. Bevorzugt beträgt die Verweilzeit maximal 28 Tage, besonders bevorzugt maximal 7 Tage und ganz besonders bevorzugt maximal 2 Tage, am meisten bevorzugt maximal 1 Tag. Maßgeblich ist die rechnerische Verweilzeit, die sich ergibt, wenn das vorgehaltene Volumen durch den Volumenstrom des Rückstandsstroms geteilt wird, der den Rückstandsbehälter durchläuft. Sofern der Zu- und/oder Ablauf von Rückstand diskontinuierlich oder mit einem nicht konstanten Volumenstrom erfolgt, können zeitlich gemittelte Werte für den Volumenstrom angesetzt werden.

Bei der Lagerung in einem Rückstandsbehälter kann es zu Ablagerungen von Rückstands-Bestandteilen an den Wandungen des Rückstandsbehälters kommen. Insbesondere im Bereich von Heiz- oder Kühlvorrichtungen besteht ein erhöhtes Risiko von Ablagerungen. Außerdem können sich Sedimente bilden, die sich am Boden des Rückstandsbehälters absetzen und zur Versumpfung führen können. Um diese Probleme zu verhindern, ist es vorteilhaft, das mindestens eine Rückstandsgemisch in mindestens einem Rückstandsbehälter zumindest zeitweise, bevorzugt durchgehend zu agitieren, bevorzugt zu rühren.

In einer weiteren bevorzugten Ausführungsform des erfindungsgemäßen Verfahrens wird im Vermischungsschritt der Rückstand B mit einer wirksamen Menge des Rückstands A abgemischt, die zu einer Stabilisierung des Rückstands B führt, wobei die Stabilisierung bevorzugt eine Erhöhung der Onset-Temperatur bezogen auf den Rückstand B alleine bedeutet. Je nach Art des Rückstands B kann dabei die wirksame Menge des Rückstands A variieren. Diese kann beispielsweise in Vorversuchen bestimmt werden, indem Mischungen des Rücktands A mit dem betreffenden Rückstand B in verschiedenen Mischungsverhältnissen hergestellt werden und dann deren thermische Stabilität mittels einer Differential Scanning Calorimetry (DSC) analog zur DIN EN ISO 11357-1:2017-02 ermittelt wird. Aus den Mischungsverhältnissen, für die eine Erhöhung der Onset-Temperatur für die exotherme Reaktion des Rückstands beobachtet wird, kann dann die wirksame Menge des Rückstands A direkt abgeleitet werden. Sofern für einen Rückstand B mehrere exotherme Reaktionen mit unterschiedlicher Onset-Temperatur auftreten, ist diejenige mit der niedrigsten Onset-Temperatur maßgeblich, deren Wärmefreisetzung den Wert von 100 J/g überschreitet.

In einer weiteren Ausführungsform des erfindungsgemäßen Verfahrens werden 10 bis 80 Gewichtsteile, bevorzugt 30 bis 75 Gewichtsteile und besonders bevorzugt 45 bis 70 Gewichtsteile des Rückstands A mit 20 bis 90 Gewichtsteilen, bevorzugt 25 bis 70 Gewichtsteilens, besonders bevorzugt 30 bis 55 Gewichtsteilen des Rückstands B vermischt, wobei jeweils der Rückstand B ein Rückstand aus der Produktion von Isophorondiisocyanat, Bis-(isocyanatomethyl)-norbornan (NBDI) und/oder 1,3- und 1,4-Bis-(isocyanatomethyl)-cyclohexan, bevorzugt aus der Produktion von Isophorondiisocyanat ist, oder es werden 5 bis 30 Gewichtsteile, bevorzugt 7 bis 20 Gewichtsteile des Rückstands A mit 70 bis 95 Gewichtsteilen, bevorzugt 80 bis 93 Gewichtsteilen des Rückstands B vermischt, wobei der jeweils der Rückstand B ein Rückstand aus der Produktion von Xylylendiisocyanat, bevorzugt von m-Xylylendiisocyanat, ist.

Das im Vermischungsschritt erhaltene Rückstandsgemisch kann beispielsweise als Zuschlagstoff zu anderen Isocyanaten verwendet werden. Aufgrund der variablen chemischen Zusammensetzung und der daraus resultierenden Eigenschaftsschwankungen ist diese Art der Verwendung jedoch limitiert. In einer bevorzugten Ausführungsform des erfindungsgemäßen Verfahrens erfolgt deshalb eine thermische Verwertung des im Vermischungsschritt erhaltenen Rückstands. Dazu kann dieser kontinuierlich oder diskontinuierlich einer Verbrennung zugeführt werden. Dabei lassen sich die durch die Vermischung erzielten vorteilhaften Eigenschaften des Rückstands nutzen. So kann der Rückstand beispielsweise bei höherer Temperatur und damit niedrigerer Viskosität gehandhabt werden, so dass beispielsweise Probleme durch Verstopfungen der Einspeise-Lanzen in der Verbrennungsanlage reduziert werden.

In einer weiteren bevorzugten Ausführungsform der Erfindung werden aus dem im Vermischungsschritt erhaltenen Rückstandsgemisch in einem nachgelagerten Verfahrensschritt monomere Diisocyanate abgetrennt. Die Abtrennung der monomeren Diisocyanate erfolgt dabei vorzugsweise unter Herstellung eines festen, bevorzugt rieselfähigen Rückstands in mindestens einem Kneter-, Schaufel-, oder Walzentrockner, bevorzugt in einem Kneter- oder Schaufeltrockner und besonders bevorzugt in einem Schaufeltrockner. Dabei wird das im Vermischungsschritt erhaltene Rückstandsgemisch gegebenenfalls zusammen mit Zuschlagstoffen in den Trockner eingebraucht und in einen gasförmigen, im Wesentlichen monomere Diisocyanate enthaltenden Teil, im Folgenden als gasförmige Mischung bezeichnet, und einen an monomeren Diisocyanaten abgereicherten Rückstand aufgetrennt. Diese Trennung erfolgt im Unterdruck bei erhöhter Temperatur, so dass sich auch hier die durch den Vermischungsschritt des erfindungsgemäßen Verfahrens erzielten vorteilhaften Eigenschaften des Rückstands ausnutzen lassen, indem beispielsweise eine höhere Temperatur angewendet werden kann.

Die in diesem Verfahrensschritt erhaltene, gasförmige Mischung aus im Wesentlichen monomeren Diisocyanaten kann kondensiert werden, wobei eine flüssige Mischung enthaltend im Wesentlichen Pentamethylendiisocyanat und mindestens ein weiteres, von Pentamethylendiisocyanat verschiedenes Diisocyanat erhalten wird. Diese flüssige Mischung kann zur Herstellung von Polyurethanen, Polythiourethanen und/oder Polyharnstoffen verwendet werden und ist ebenfalls Gegenstand der vorliegenden Erfindung. Bei Bedarf kann die flüssige Mischung vor der Herstellung der Polyurethane, Polythiourethane und oder Polyharnstoffe weiteren Reinigungsschritten, wie beispielsweise Destillation, Filtration, Kristallisation und/oder Extraktion, unterzogen werden und/oder mit Additiven versetzt werden. Bevorzugt ist das mindestens eine weitere, von Pentamethylendiisocyanat verschiedene Diisocyanat in der flüssigen Mischung Hexamethylendiisocyanat, Diisocyanatohexylmethan, Isophorondiisocyanat (IPDI), Bis-(isocyanatomethyl)-norbornan (NBDI), 1,3- und 1,4-Bis-(isocyanatomethyl)-cyclohexan und/oder Xylylyendiisocyanat, besonders bevorzugt Isophorondiisocyanat und/oder Xylylendiisocyanat und ganz besonders bevorzugt Isophorondiisocyanat und/oder m-Xylylendiisocyanat. Da die über das erfindungsgemäße Verfahren zurückgewonnene gasförmige Mischung und die erfindungsgemäße flüssige Mischung aus Rückständen aus der Produktion organischer Isocyanate durch Phosgenierung organischer Amine stammen, enthalten sie geringe Mengen an zusätzlichen Verbindungen, die nicht in den bereits bekannten direkt hergestellten Verfahrensprodukten vorhanden sind. Im Zusammenhang mit der erfindungsgemäßen flüssigen Mischung ist der Begriff "im Wesentlichen" so zu verstehen, dass vorzugsweise 95 Gew.-% oder mehr, besonders bevorzugt 97 Gew.-% oder mehr und ganz besonders bevorzugt 99 Gew.-% oder mehr, bezogen auf die Gesamtmenge der Mischung, Pentamethylendiisocyanat und mindestens ein weiteres, von Pentamethylendiisocyanat verschiedenes Diisocyanat sind.

Ein weiterer Gegenstand der vorliegenden Erfindung ist eine gasförmige oder flüssige Mischung, erhältlich oder hergestellt nach dem erfindungsgemäßen Verfahren. Die gasförmige Mischung oder gegebenenfalls die flüssige Mischung kann in einem weiteren Verfahrensschritt bei Bedarf destillativ aufgearbeitet werden, um monomeres Pentamethylendiisocyanat und das mindestens eine von Pentamethylendiisocyanat verschiedene Diisocyanat zu isolieren.

Ein weiterer Gegenstand der vorliegenden Erfindung ist die Verwendung eines ersten Rückstands A aus der Produktion von 1,5-Pentandiisocyanat zur Stabilisierung eines zweiten Rückstands B aus der Produktion mindestens eines von 1,5-Pentandiisocyanat verschiedenen, organischen Isocyanats, wobei die Stabilisierung bevorzugt eine Erhöhung der Onset-Temperatur bezogen auf den Rückstand B alleine bedeutet.

Die Onset-Temperaturen der einzelnen Rückstände bzw. verschiedener Rückstandsmischungen können beispielsweise in Vorversuchen mittels Differential Scanning Calorimetry (DSC) analog zur DIN EN ISO 11357-1:2017-02 ermittelt werden. Entscheidend ist dabei die jeweilige Onset-Temperatur der exothermen Reaktion des Isocyanat-Rückstands. Sofern für einen Rückstand mehrere exotherme Reaktionen mit unterschiedlicher Onset-Temperatur beobachtet werden, ist diejenige mit der niedrigsten Onset-Temperatur maßgeblich, deren Wärmefreisetzung den Wert von 100 J/g überschreitet.

In einer weiteren bevorzugten Ausführungsform der erfindungsgemäßen Verwendung stammt der zweite Rückstand B aus der Produktion mindestens eines von 1,5-Pentandiisocyanat verschiedenen, aliphatischen, cycloaliphatischen oder araliphatischen Diisocyanats, vorzugsweise aus der Produktion eines araliphatischen oder cycloaliphatischen Diisocyanats, besonders bevorzugt aus der Produktion von Hexamethylendiisocyanat, Diisocyanatohexylmethan, Isophorondiisocyanat, Bis-(isocyanatomethyl)-norbornan (NBDI), 1,3- und 1,4-Bis-(isocyanatomethyl)-cyclohexan und/oder Xylylendiisocyanat, ganz besonders bevorzugt aus der Produktion von IPDI und/oder Xylylendiisocyanat und am meisten bevorzugt aus der Produktion von IPDI und/oder XDI.

In einer weiteren bevorzugten Ausführungsform der erfindungsgemäßen Verwendung sind der Rückstand A und/oder der Rückstand B, bevorzugt der Rückstand A und der Rückstand B, Destillationsrückstände. Die erfindungsgemäße Verwendung bietet sich vor allem für Destillationsrückstände an, weil sich dann in den vorgelagerten Destillationsschritten entsprechende Optimierungen durchführen lassen. So kann beispielsweise der Restmonomergehalt im Sumpfprodukt der Destillationskolonne gesenkt werden, so dass ein geringerer Verlust an Wertstoff auftritt, oder es kann ein Teil der Kühlleistung eingespart werden, die erforderlich ist, um die Rückstände auf ihre jeweils zulässigen Lagertemperaturen herunterzukühlen.

Ein weiterer Gegenstand der vorliegenden Erfindung ist eine stabilisierte Rückstandsmischung, enthaltend 10 bis 80 Gew.-% eines ersten Rückstands A aus der Produktion von 1,5-Pentandiisocyanat und 20 bis 90 Gew.-% eines Rückstands B aus der Produktion von Isophorondiisocyanat, Bis-(isocyanatomethyl)-norbornan (NBDI) oder 1,3- und 1,4-Bis-(isocyanatomethyl)-cyclohexan, bevorzugt von Isophorondiisocyanat oder enthaltend 5 bis 30 Gew.-% eines ersten Rückstands A aus der Produktion von 1,5-Pentandiisocyanat und 70 bis 95 Gew.-% eines zweiten Rückstands B aus der Produktion von Xylylendiisocyanat, bevorzugt von m-Xylylendiisocyanat.

Hierbei bedeutet die Stabilisierung bevorzugt eine Erhöhung der Onset-Temperatur bezogen auf den Rückstand B alleine. Je nach Art des Rückstands B kann dabei die wirksame Menge des Rückstands A variieren. Diese kann beispielsweise in Vorversuchen bestimmt werden, indem Mischungen des Rücktands A mit dem betreffenden Rückstand B in verschiedenen Mischungsverhältnissen hergestellt werden und dann deren thermische Stabilität in einer Differential Scanning Calorimetry (DSC) analog zur DIN EN ISO 11357-1:2017-02 ermittelt wird. Aus den Mischungsverhältnissen, für die eine Erhöhung der Onset-Temperatur für die exotherme Reaktion des Rückstands beobachtet wird, kann dann die wirksame Menge des Rückstands A direkt abgeleitet werden. Sofern für einen Rückstand B mehrere exotherme Reaktionen mit unterschiedlicher Onset-Temperatur auftreten, ist diejenige mit der niedrigsten Onset-Temperatur maßgeblich.

### Beispiele

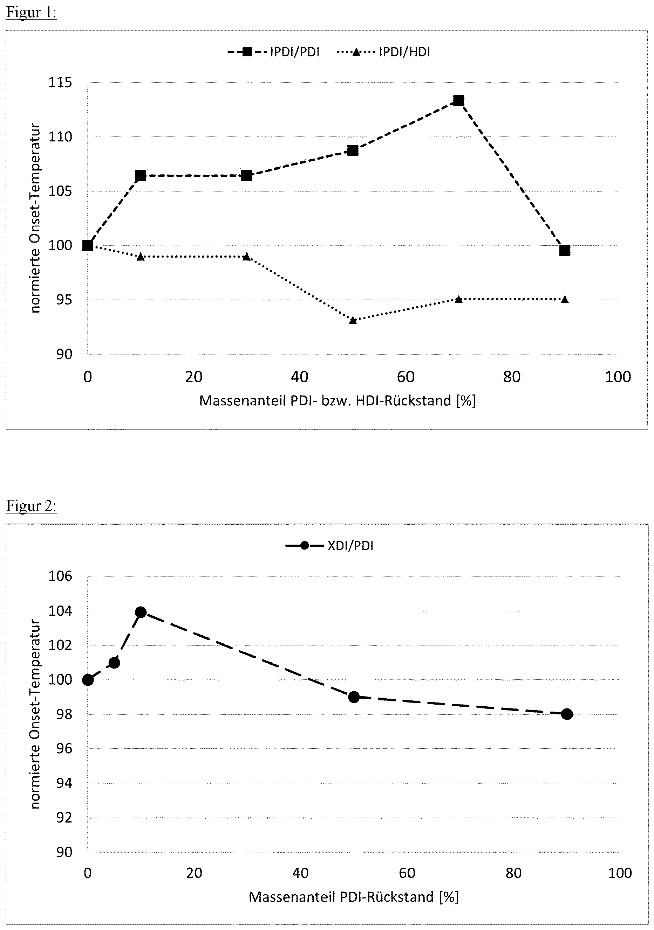

Die vorliegende Erfindung wird im Folgenden anhand der Figur 1 und Figur 2 sowie der nachfolgenden Beispielen erörtert, ist jedoch nicht auf diese beschränkt.

Für die Differential Scanning Calorimetry (DSC) wurden jeweils einige Milligramm der Rückstandsmischung unter Stickstoffatmosphäre in einen vergoldeten Stahltiegel eingewogen und dieser mit Hilfe einer Tiegelpresse verschlossen. Die so präparierten Proben wurden dann in der DSC Messapparatur gegen einen Referenztiegel vermessen, welcher mit inertem Aluminiumoxid befüllt war. Die Messapparatur entsprach dabei den Anforderungen der DIN EN ISO 11357-1:2017-02. Beide Tiegel wurden mit einer Heizrate von 3K/min aufgeheizt (sogenannte Screening DSC) und es wurde die Differenz des Wärmestroms in den Tiegel mit der Rückstandsmischung und des Wärmestroms in den Referenztiegel aufgezeichnet. Entsprechend der DIN EN ISO 11357-1:2017-02 ist bei einem exothermen Peak, also einer Umwandlung mit Wärmefreisetzung, der Wärmestrom in den Probekörpertiegel geringer als der in den Referenztiegel und die aufgezeichnete Kurve durchläuft entsprechend ein Minimum. Zur Beurteilung der thermischen Stabilität wurde die Onset-Temperatur des ersten exothermen Peaks ermittelt, dessen Wärmefreisetzung den Wert von 100 J/g überschritt, also die die Temperatur, bei der die aufgezeichnete Wärmestromdifferenz begann, sich von der Basislinie des Probekörpers zu unterscheiden.

### Beispiel 1

Ein Rückstand aus einer Destillation von Isophorondiisocyanat (IPDI) wurde in verschiedenen Mischungsverhältnissen mit einem Rückstand aus einer Destillation von Pentamethylendiisocyanat (PDI) gemischt. Die Mischungsverhältnisse von IPDI-Massenanteil:PDI-Massenanteil in den Rückstandsmischungen betrugen 100:0, 90:10, 70:30, 50:50, 30:70 und 10:90. Die Mischungen wurden anschließend mittels Differenz-Thermoanalyse untersucht und die Onset-Temperaturen für die exotherme Reaktion der jeweiligen Rückstandsmischung wurde ermittelt.

### Beispiel 2

Ein Rückstand aus einer Destillation von Isophorondiisocyanat (IPDI) wurde in verschiedenen Mischungsverhältnissen mit einem Rückstand aus einer Destillation von Hexamethylendiisocyanat (HDI) gemischt. Die Mischungsverhältnisse von IPDI-Massenanteil:HDI-Massenanteil in den Rückstandsmischungen betrugen 100:0, 90:10, 70:30, 50:50, 30:70 und 10:90). Die Mischungen wurden anschließend mittels Differenz-Thermoanalyse untersucht und die Onset-Temperaturen für die exotherme Reaktion der jeweiligen Rückstandsmischung wurde ermittelt.

### Diskussion von Beispiel 1 und 2:

Die Onset-Temperaturen aus Beispiel 1 und Beispiel 2 wurden jeweils normiert, so dass die 100:0-Mischung, also ein Destillationsrückstand aus einer IPDI-Destillation den Wert 100 erhielt. Die so normierten Onset-Temperaturen sind in Figur 1 aufgetragen.

Im Diagramm der Figur 1 wurde die normierte Onset-Temperatur auf der Y-Achse und der Massenanteil PDI- bzw. HDI-Rückstand auf der X-Achse aufgetragen. Der Graph mit den quadratischen Messpunkten stellt das Ergebnis von Beispiel 1 (IPDI/PDI) dar und der Graph mit den dreieckigen Messpunkten stellt das Ergebnis von Beispiel 2 (IPDI/HDI) dar.

Aus den Graphen in Figur 1 ist erkennbar, dass die Mischung mit PDI im Bereich von ca. 10 Gew.-% PDI-Anteil bis ca. 80 Gew.-% PDI-Anteil zu einer Erhöhung der Onset-Temperatur und damit zu einer Stabilisierung der Rückstandsmischung führt, während durch Beimengung von HDI keine der Testmischungen eine normierte Onset-Temperatur >100 aufweist. Vielmehr durchläuft die normierte Onset-Temperatur ein Minimum, die Beimengung von HDI-Rückstand führt also zu einer Destabilisierung der IPDI-Rückstandsmischung.

### Beispiel 3

Ein Rückstand aus einer Destillation von m-Xylylendiisocyanat (XDI) wurde in verschiedenen Mischungsverhältnissen mit einem Rückstand aus einer Destillation von Pentamethylendiisocyanat gemischt. Die Mischungsverhältnisse von XDI-Anteil:PDI-Anteil in den Rückstandsmischungen betrugen 100:0, 95:5 90:10, 50:50 und 10:90) Die Mischungen wurden anschließend mittels Differenz-Thermoanalyse untersucht. Die normierten Onset-Temperaturen (Onset-Temperatur des reinen XDI-Rückstands auf den Wert 100 normiert) sind in Figur 2 aufgetragen.

Im Diagramm der Figur 2 wurde die normierte Onset-Temperatur auf der Y-Achse und der Massenanteil PDI-Rückstand auf der X-Achse aufgetragen. Der Graph mit den runden Messpunkten stellt das Ergebnis von Beispiel 3 (XDI/PDI) dar.

Aus den Graphen in Figur 2 ist erkennbar, dass die Mischung mit PDI-Rückstand im Bereich von ca. 5 Gew.-% PDI-Anteil bis ca. 30 Gew.-% PDI-Anteil zu einer Erhöhung der normierten Onset-Temperatur und damit zu einer Stabilisierung der Rückstandsmischung führt.

## Patentansprüche

1. Verfahren zur Behandlung von Rückständen aus der Produktion organischer Isocyanate durch Phosgenierung organischer Amine, umfassend einen Vermischungsschritt eines ersten Rückstands A mit einem zweiten Rückstand B unter Erhalt einer Rückstandsmischung, wobei der Rückstand A aus der Produktion von 1,5-Pentandiisocyanat erhalten wurde und der Rückstand B aus der Produktion eines von 1,5-Pentandiisocyanat verschiedenen Isocyanats stammt.

2. Verfahren gemäß Anspruch 1, **dadurch gekennzeichnet, dass** der Rückstand A und/oder der Rückstand B, bevorzugt der Rückstand A und der Rückstand B, Destillationsrückstände sind.

3. Verfahren gemäß Anspruch 1 oder 2, **dadurch gekennzeichnet, dass** der Rückstand B aus der Produktion mindestens eines von 1,5-Pentandiisocyanat verschiedenen aliphatischen, cycloaliphatischen oder araliphatischen Diisocyanats, vorzugsweise mindestens eines araliphatischen oder cycloaliphatischen Diisocyanats stammt.

4. Verfahren gemäß einem der Ansprüche 1 bis 3, **dadurch gekennzeichnet, dass** der Rückstand B aus der Produktion von Hexamethylendiisocyanat, Diisocyanatohexylmethan, Isophorondiisocyanat, Bis-(isocyanatomethyl)-norbornan, 1,3- und 1,4-Bis-(isocyanatomethyl)-cyclohexan und/oder Xylylyendiisocyanat, bevorzugt aus der Produktion von Isophorondiisocyanat und/oder Xylylendiisocyanat und besonders bevorzugt aus der Produktion von Isophorondiisocyanat und/oder m-Xylylendiisocyanat stammt.

5. Verfahren gemäß einem der Ansprüche 1 bis 4, **dadurch gekennzeichnet, dass** im Vermischungsschritt zumindest zeitweise, bevorzugt durchgehend eine Temperatur zwischen 15 und 180 °C, bevorzugt zwischen 40 °C und 150 °C und besonders bevorzugt zwischen 60 °C und 110 °C vorliegt.

6. Verfahren gemäß einem der Ansprüche 1 bis 5, **dadurch gekennzeichnet, dass** durch den Vermischungsschritt mindestens ein Rückstandsgemisch erhalten wird, welches in mindestens einem Rückstandsbehälter gelagert wird, wobei der Vermischungsschritt in einer separaten, dem Rückstandsbehälter vorgelagerten Mischvorrichtung oder vorzugsweise in dem Rückstandsbehälter selbst erfolgen kann.

7. Verfahren gemäß Anspruch 6, **dadurch gekennzeichnet, dass** die Lagerung zumindest zeitweise, bevorzugt durchgehend bei einer Temperatur zwischen 15 °C und 180 °C, bevorzugt zwischen 40 °C und 150 °C und besonders bevorzugt zwischen 60 °C und 110 °C erfolgt.

8. Verfahren gemäß Anspruch 6 oder 7, **dadurch gekennzeichnet, dass** das mindestens eine Rückstandsgemisch in mindestens einem Rückstandsbehälter zumindest zeitweise, bevorzugt durchgehend agitiert, bevorzugt gerührt wird.

9. Verfahren gemäß einem der Ansprüche 1 bis 8, **dadurch gekennzeichnet, dass** der Vermischungsschritt von einer wirksamen Menge des Rückstands A mit dem Rückstand B zu einer Stabilisierung des Rückstands B führt, wobei die Stabilisierung bevorzugt eine Erhöhung der Onset-Temperatur bezogen auf den Rückstand B alleine bedeutet.

10. Verfahren gemäß einem der Ansprüche 1 bis 9, **dadurch gekennzeichnet, dass** 10 bis 80 Gewichtsteile des Rückstands A mit 20 bis 90 Gewichtsteilen des Rückstands B vermischt werden, wobei der Rückstand B aus der Produktion von Isophorondiisocyanat, Bis-(isocyanatomethyl)-norbornan (NBDI) und/oder 1,3- und 1,4-Bis-(isocyanatomethyl)-cyclohexan, bevorzugt von Isophorondiisocyanat ist, oder dass 5 bis 30 Gewichtsteile des Rückstands A mit 70 bis 95 Gewichtsteilen des Rückstands B vermischt werden, wobei der Rückstand B aus der Produktion von Xylylendiisocyanat, bevorzugt von m-Xylylendiisocyanat ist.

11. Verfahren gemäß einem der Ansprüche 1 bis 10, **dadurch gekennzeichnet, dass** der Rückstand kontinuierlich oder diskontinuierlich einer Verbrennung zugeführt wird.

12. Verwendung eines ersten Rückstands A aus der Produktion von 1,5-Pentandiisocyanat zur Stabilisierung eines zweiten Rückstands B aus der Produktion mindestens eines von 1,5-Pentandiisocyanat verschiedenen, organischen Isocyanats, wobei die Stabilisierung bevorzugt eine Erhöhung der Onset-Temperatur bezogen auf den Rückstand B alleine bedeutet.

13. Verwendung gemäß Anspruch 12, **dadurch gekennzeichnet, dass** der zweite Rückstand B aus der Produktion mindestens eines von 1,5-Pentandiisocyanat verschiedenen, aliphatischen, cycloaliphatischen oder araliphatischen Diisocyanats, vorzugsweise aus der Produktion mindestens eines araliphatischen oder cycloaliphatischen Diisocyanats, besonders bevorzugt aus der Produktion von Hexamethylendiisocyanat, Diisocyanatohexylmethan, Isophorondiisocyanat, Bis-(isocyanatomethyl)-norbornan (NBDI), 1,3- und 1,4-Bis-(isocyanatomethyl)-cyclohexan und/oder Xylylendiisocyanat, ganz besonders bevorzugt aus der Produktion von Isophorondiisocyanat und/oder Xylylendiisocyanat und am meisten bevorzugt aus der Produktion von Isophorondiisocyanat und/oder m-Xylylendiisocyanat stammt.

14. Verwendung gemäß Anspruch 12 oder 13, **dadurch gekennzeichnet, dass** der Rückstand A und/oder der Rückstand B, bevorzugt der Rückstand A und der Rückstand B, Destillationsrückstände sind.

15. Stabilisierte Rückstandsmischung, enthaltend 10 bis 80 Gew.-% eines ersten Rückstands A aus der Produktion von 1,5-Pentandiisocyanat und 20 bis 90 Gew.-% eines Rückstands B aus der Produktion von Isophorondiisocyanat, Bis-(isocyanatomethyl)-norbornan (NBDI) oder 1,3- und 1,4-Bis-(isocyanatomethyl)-cyclohexan, bevorzugt von Isophorondiisocyanat oder enthaltend 5 bis 30 Gew.-% eines ersten Rückstands A aus der Produktion von 1,5-Pentandiisocyanat und 70 bis 95 Gew.-% eines zweiten Rückstands B aus der Produktion von Xylylendiisocyanat, bevorzugt von m-Xylylendiisocyanat.
